(19) Europäisches Patentamt
European Patent Office
Office européen des brevets

(11) **EP 1 413 565 B1**

(12) **EUROPÄISCHE PATENTSCHRIFT**

(45) Veröffentlichungstag und Bekanntmachung des Hinweises auf die Patenterteilung:
**30.09.2009 Patentblatt 2009/40**

(51) Int Cl.:
***C07B 57/00*** *(2006.01)* ***C07C 255/24*** *(2006.01)*

(21) Anmeldenummer: **03022897.7**

(22) Anmeldetag: **09.10.2003**

(54) **Verfahren zur Racematsspaltung von 3-Aminopentannitril**

Method for optical resolution of 3-aminopentanenitrile

Procédé pour la résolution optique du 3-aminopentanonitrile

(84) Benannte Vertragsstaaten:
**AT BE BG CH CY CZ DE DK EE ES FI FR GB GR HU IE IT LI LU MC NL PT RO SE SI SK TR**

(30) Priorität: **22.10.2002 DE 10249339**

(43) Veröffentlichungstag der Anmeldung:
**28.04.2004 Patentblatt 2004/18**

(73) Patentinhaber: **Saltigo GmbH**
**40764 Langenfeld (DE)**

(72) Erfinder:
• **Dreisbach, Claus, Dr.**
**42799 Leichlingen (DE)**
• **Schlummer, Björn, Dr.**
**51377 Leverkusen (DE)**

(74) Vertreter: **Wichmann, Birgid et al**
**LANXESS Deutschland GmbH**
**LIP-IPR**
**Q 18 / 1450**
**51369 Leverkusen (DE)**

(56) Entgegenhaltungen:
WO-A-20/04002924    US-A- 3 808 254
US-A- 4 072 698    US-A- 4 260 556

• JACQUES, JEAN ET AL.: "Enantiomers, Racemates, and Resolutions" 1981, WILEY-INTERSCIENCE PUBLICATION , NEW YORK , XP002292433 * Seite 253 - Seite 256 * * Seite 259 - Seite 261 * * Seite 396 - Seite 399 *
• WILLIAMS, PHILIP G. ET AL.: J. NAT. PROD., Bd. 65, 2002, Seiten 29-31, XP002292432
• ELVERS, B. ET AL, EDITORS: "Ullmann's Encyclopedia of Industrial Chemistry, 5. Edition, Volume A18" 1991, VCH VERLAGSGESELLSCHAFT , WEINHEIM * Seite 182 - Seite 183 *

EP 1 413 565 B1

## Beschreibung

Die vorliegende Erfindung betrifft ein Verfahren zur Spaltung von racemischem 3-Aminopentannitril unter Gewinnung von enantiomerenangereichertem 3-Aminopentannitril oder dessen Salzen durch Umsetzung von racemischem 3-Aminopentannitril mit ausgewählten enantiomerenangereicherten N-geschützten Aminosäuren.

(R)-3-Aminopentannitril und seine Salze sind wichtige Ausgangsmaterialien zur Synthese von pharmazeutischen Wirkstoffen. Da die pharmazeutische Wirkung häufig auf nur ein Enantiomer zurückzuführen ist, ist es wünschenswert, dieses gewünschte Enantiomer in hoher optischer Reinheit herzustellen bzw. bereitzustellen. Ziel ist es daher, die Synthese so zu planen, dass racemische Zwischenverbindungen in ihre Enantiomere getrennt werden, um zu homochiralen Produkten zu gelangen.

In US-A-4,260,556 wird die Darstellung von racemischem 3-Aminopentannitril durch Reaktion von 2-Pentennitril mit Ammoniak in Anwesenheit eines metallischen Katalysators beschrieben.

In US-A-4,496,474 wird die Umsetzung von 2-Pentennitril mit Alkylaminen unter Bildung der entsprechenden racemischen Cyanoverbindungen offenbart. In EP-A-0 449 297 wird ferner eine verbesserte Variante dieser prinzipiellen Umsetzung von 2-Pentennitril mit Alkylaminen in Anwesenheit von 15-60 Gewichtsprozent Wasser beschrieben.

US-A-5,902,883 beschreibt einen Prozess zur Cyanobutylierung von Ammoniak, Alkylaminen oder Hydrazin mit 3- und 4-Pentennitril, bzw. Gemischen derselben, unter Bildung der racemischen Alkylaminonitrile.

Im vorgenannten Stand der Technik wird keinerlei Hinweis auf eine Trennung der jeweils erhaltenen racemischen Gemische gegeben.

Die Racematspaltung von Aminen durch Bildung diastereomerer Salze mit chiralen Säuren ist aus Jacques, Jean et al.:"Enantiomers, Racemates, and Resolutions", 1981, Wiley-Interscience Publication, S. 252 - 399, bekannt.

Die Racematspaltung von 2-Aminopentannitrilen durch Kristallisation ihrer diastereomeren Salze mit optisch aktiven Säuren ist aus US-A-4072698 bekannt. WO 2004/002924 A beschreibt ein Verfahren zur Racematspaltung von 3-Aminopentannitrilen durch Kristallisation mit chiralen Säuren, insbesondere mit Weinsäurederivaten.

In J. Nat. Prod. 65 (2002), 29-31 wird die Darstellung von enantiomerenreinem (R)- und (S)-3-Aminopentannitril durch aufbauende Synthese ausgehend von (R)- bzw. (S)-2-Aminobutanol beschrieben.

Die Darstellung von enantiomerenreinem bzw. -angereichertem (R)- oder (S)-3-Aminopentannitril durch Spaltung des racemischen 3-Aminopentannifrils mit ausgewählten N-geschützten Aminosäuren ist bisher im Stand der Technik nirgends beschrieben.

Da die einzige, aus J. Nat. Prod. 65 (2002), 29-31 bekannte Methode zur Synthese enantiomerenangereicherten 3-Aminopentannitrils sehr aufwändig ist, bestand die Aufgabe der vorliegenden Erfindung darin, ein geeignetes Verfahren zur Spaltung von racemischem 3-Aminopentannitril unter Anreicherung eines der beiden Enantiomere zur Verfügung zu stellen.

Gegenstand der Erfindung ist ein Verfahren zur Gewinnung von enantiomerenangereichertem 3-Aminopentannitril oder dessen Salzen aus racemischem 3-Aminopentannitril durch

1) Umsetzung von racemischem 3-Aminopentannitril mit einer enantiomerenangereicherten organischen Säure unter Bildung zweier diastereomerer Salze aus dem 3-Aminopentannitril und der organischen Säure,
2) Abtrennung eines diastereomeren Salzes aus dem Reaktionsgemisch und
3) Überführung dieses diastereomeren Salzes in das enantiomerenangereicherte 3-Aminopentannitril oder dessen Salze,

wobei die enantiomerangereicherte organische Säure (L)-N-Boc-alanin, (L)-N-Bocasparaginsäure, (L)-N-Boc-Histidin, (L)-N-Boc-Isoleucin, (L)-N-Boc-Leucin, (L)-N-Boc-Methionin, (L)-N-Boc-phenylalanin, (L)-N-Boc-Prolin, (L)-N-Boc-Serin, (L)-N-Boc-Threonin, (L)-N-Boc-Tyrosin oder (L)-N-Boc-Valin, oder ihre jeweiligen Enantiomere ist.

Enantiomerenangereichertes 3-Aminopentannitril im Sinne der Erfindung bedeutet die enantiomerenreinen Verbindungen (R)-3-Aminopentannitril und (S)-3-Aminopentannitril oder Mischungen der beiden Enantiomere (R)-3-Aminopentannitril und (S)-3-Aminopentannitril, in denen ein Enantiomer in einem Enantiomerenüberschuss, im Folgenden auch ee ("enantiomeric excess") genannt, im Vergleich zum anderen Enantiomer vorliegt. Bevorzugt beträgt dieser Enantiomerenüberschuss 2 bis 100 % ee, besonders bevorzugt 60 % bis 100 % und ganz besonders bevorzugt 85 bis 100 %. Eine Definition des ee-Werts wird im Rahmen der Beispiele dieser Anmeldung angegeben.

Als enantiomerenangereicherte organische Säuren im Sinne dieser Erfindung werden die enantiomerenangereicherten.

Unterstreichung wegnehmen N-geschützten Aminosäurederivate (L)-N-Boc-alanin, (L)-N-Boc-asparaginsäure, (L)-N-Boc-Histidin, (L)-N-Boc-Isoleucin, (L)-N-Boc-Leucin, (L)-N-Boc-Methionin, (L)-N-Boc-phenylalanin, (L)-N-Boc-Prolin, (L)-N-Boc-Serin, (L)-N-Boc-Threonin, (L)-N-Boc-Tyrosin und (L)-N-Boc-valin eingesetzt.

Sinngemäß lassen sich auch alle Enantiomeren der zuvor genannten speziellen Carbonsäuren einsetzen.

Besonders bevorzugte Carbonsäuren zur Gewinnung des (R)-enantiomerenangereicherten 3-Aminopentannitrils sind (L)-N-Boc-Isoleucin und (L)-N-Boc-Serin. Die bei der Abtrennung des diastereomeren Salzes in Schritt 2) des erfindungsgemäßen Verfahrens anfallende Mutterlauge kann weiter aufgearbeitet werden und liefert dann entsprechend das (S)-enantiomerenangereicherte 3-Aminopentannitril oder dessen Salze.

Derartige enantiomerenangereicherte organische Carbonsäuren, sind käuflich erhältlich. Der Begriff "enantiomerenangereicherte organische Säure/Carbonsäure" bedeutet im Sinne dieser Erfindung die jeweiligen enantiomerenreinen organischen Säuren/Carbonsäuren oder Mischungen der jeweiligen Enantiomere, in denen ein Enantiomer in einem Enantiomerenüberschuss, im Folgenden auch ee ("enantiomeric excess") genannt, im Vergleich zum anderen Enantiomer vorliegt. Bevorzugt beträgt dieser ee-Wert 10-100 %, besonders bevorzugt 60-100 % und ganz besonders bevorzugt 95-100 %.

Die Umsetzung des racemischen 3-Aminopentannitrils mit der enantiomerenangereicherten organischen Säure unter Bildung zweier diastereomerer Salze in Schritt 1) des erfindungsgemäßen Verfahrens wird zweckmäßigerweise in einem Temperaturbereich von 0°C bis zur Zersetzungstemperatur der Reaktionsteilnehmer, vorzugsweise in einem Bereich von 20 bis 120°C durchgeführt. Besonders bevorzugt erfolgt die Umsetzung in einem Bereich von 60 bis 80°C.

Die Umsetzung in Schritt 1) erfolgt üblicherweise in Gegenwart eines Lösungsmittels. Als Lösungsmittel können polare oder unpolare organische Lösungsmittel, beispielsweise Ether, bevorzugt Dialkylether, insbesondere Diethylether und *tert*-Butylmethylether, oder cyclische Ether, insbesondere Tetrahydrofuran, geradkettige oder verzweigte $C_1$-$C_{10}$-Alkohole, bevorzugt Methanol, Ethanol, n-Propanol oder iso-Propanol, geradkettige oder verzweigte $C_1$-$C_{10}$-Alkoholderivate, wie teilhalogenierte $C_1$-$C_{10}$-Alkohole, einkernige und mehrkernige aromatische carbocyclische und heterocyclische Kohlenwasserstoffe, bevorzugt Toluol, aliphatische $C_1$-$C_{10}$ Kohlenwasserstoffe sowie funktionalisierte Kohlenwasserstoffe wie Nitrile, beispielsweise Acetonitril, Carbonsäureester, bevorzugt Essigsäureethylester, Ketone und Halogenverbindungen verwendet werden. Die Menge des eingesetzten Lösungsmittels ist unkritisch.

Die Reaktionsteilnehmer, d.h. die enantiomerenangereicherte organische Säure und das racemische 3-Aminopentannitril, können in beliebigen Mengen eingesetzt werden. Bevorzugt werden 0.1 - 1 Äquivalente der organischen Säure, besonders bevorzugt etwa 0.5 Äquivalente der organischen Säure eingesetzt.

Durch die Umsetzung des racemischen 3-Aminopentannitrils mit der enantiomerenangereicherten organischen Säure werden im erfindungsgemäßen Verfahren zwei diastereomere Salze des 3-Aminopentannitrils und der organischen Säure gebildet. Hierbei handelt es sich beispielsweise um die diastereomeren Salze

(R)-3-Aminopentannitril-N-Boc-L-isoleucinat,
(R)-3-Aminopentannitril-N-Boc-L-leucinat,
(R)-3-Aminopentannitril-N-Boc-L-serinat,
und ihrer jeweiligen Enantiomere.

Bei der Umsetzung in Schritt 1) fällt üblicherweise eines der beiden diastereomeren Salze in Form eines Feststoffes aus. Die Abtrennung dieses diastereomeren Salzes aus dem Reaktionsgemisch erfolgt in Schritt 2) üblicherweise durch Abfiltrieren oder Abzentrifugieren.

Das nach dem Abfiltrieren des diastereomeren Salzes verbleibende Reaktionsgemisch kann sofern gewünscht zur Gewinnung des zweiten diastereomeren Salzes aufgearbeitet werden.

In einer alternativen Ausführungsform des erfindungsgemäßen Verfahrens kann es zweckmäßig sein, das in Schritt 2) als Feststoff abgetrennte diastereomere Salz einer oder mehreren Umkristallisationen zu unterwerfen, bevor in Schritt 3) des Verfahrens das enantiomerenangereicherte 3-Aminopentannitril oder dessen Salze gewonnen werden. Für diese Umkristallisationen hat sich die Verwendung der bereits für Schritt 1) genannten Lösungsmitteln bewährt.

In Schritt 3) wird anschließend aus dem abgetrennten diastereomeren Salz das gewünschte enantiomerenangereicherte 3-Aminopentannitril oder dessen Salze gewonnen. Dies kann auf chemischem Wege erfolgen.

Hierzu wird das diastereomere Salz mit einer Base umgesetzt, welche stärker ist als die Aminfunktion im 3-Aminopentannitril, z.B. mit Alkalihydroxiden, Erdalkalihydroxiden, bevorzugt Natrium- oder Kaliumhydroxid, Alkalicarbonaten oder Erdalkalicarbonaten, bevorzugt Natrium- oder Kaliumcarbonat, und anschließend extrahiert mit einem organischen Lösungsmittel. Als Lösungsmittel können die zuvor bereits genannten organischen Lösungsmittel eingesetzt werden. Zweckmäßigerweise werden 1-5 N Lösungen der Base bei einer Temperatur von 0 bis 100°C eingesetzt. Besonders bevorzugt ist die Verwendung von 1N Natronlauge bei einer Temperatur von 15 bis 40°C mit anschließender Extraktion durch Dichlormethan. Bei dieser Umsetzung des diastereomeren Salzes mit einer Base wird das enantiomerenangereicherte 3-Aminopentannitril als solches erhalten, d.h. als freie Base.

Alternativ dazu kann das diastereomere Salz auch in einem organischen Lösungsmittel mit einer starken Säure umgesetzt werden, die stärker ist als die in Schritt 1) eingesetzte enantiomerenangereicherte organische Säure. Hierbei wird beispielsweise Salzsäure, Jodwasserstoffsäure, Bromwasserstoffsäure, Flusssäure, Schwefelsäure, Salpetersäure, Perchlorsäure, Phosphorsäure oder Methansulfonsäure eingesetzt. Bevorzugt sind Salzsäure, Bromwasserstoffsäure, Schwefelsäure und Methansulfonsäure. Diese Umsetzung in Schritt 3) wird bei einer Temperatur im Bereich von 0°C bis etwa zur Zersetzungstemperatur der Reaktionsteilnehmer durchgeführt, bevorzugt in einem Bereich von 20 bis 150°C und besonders bevorzugt in einem Bereich von 40 bis 90°C. Als Lösungsmittel können die zuvor bereits genannten organischen Lösungsmittel eingesetzt werden. Bevorzugt werden Ether, besonders bevorzugt *tert*-Butylmethylether, als Lösungsmittel eingesetzt. Bei dieser Variante wird das gewünschte enantiomerenangereicherte 3-Aminopentannitril direkt als Salz der starken Säure erhalten. Erhalten werden z.B. die folgenden Salze des 3-Aminopentannitrils:

enantiomerenangereichertem 3-Aminopentannitrilfluorid
enantiomerenangereichertem 3-Aminopentannitrilchlorid
enantiomerenangereichertem 3-Aminopentannitrilbromid
enantiomerenangereichertem 3-Aminopentannitriljodid
enantiomerenangereichertem 3-Aminopentannitrilsulfat
enantiomerenangereichertem 3-Aminopentannitrilhydrogensulfat
enantiomerenangereichertem 3-Aminopentannitrilphosphat
enantiomerenangereichertem 3-Aminopentannitrilhydrogenphosphat
enantiomerenangereichertem 3-Aminopentannitrildihydrogenphosphat
enantiomerenangereichertem 3-Aminopentannitrilnitrat
enantiomerenangereichertem 3-Aminopentannitrilperchlorat.

Diese Salze können zur Herstellung pharmazeutischer Wirkstoffe verwendet werden.

**Beispiele:**

In allen nachfolgenden Beispielen wird die optische Reinheit des (R)- bzw. (S)-3-Aminopentannitrils durch Gaschromatographie an einem chiralen Säulenmaterial (gamma-Dex 225) bestimmt und über den nachfolgend definierten ee ("enantiomeric excess") (S) oder (R)-Wert angegeben.
Der ee-Wert ergibt sich dabei über folgende Formeln:

$$ee\ (S) = \frac{m(S)-m(R)}{m\ (S+R)} \times 100\%$$

$$ee\ (R) = \frac{m(R)-m(S)}{m\ (S+R)} \times 100\%$$

wobei ee (S) bzw. ee (R) die optische Reinheit des Enantiomers S bzw. R ist, m(S) die Stoffmenge des Enantiomers S und m(R) die Stoffmenge des Enantiomers R ist. (Beispiele: Für ein Racemat gilt: R=S => ee = 0; für die reine (S)-Form gilt: ee (S) = 100 %; für ein Verhältnis von S:R = 9:1 ist ee (S) = 80 %)

**Vergleichsbeispiel 1:**

**Racematspaltung unter Verwendung von (-)-Diaceton-2-keto-L-gulonsäure**

1.50 g (15.3 mmol) 3-Aminopentannitril werden in der in Tabelle 1a angegebenen Menge des jeweiligen Lösungsmittels gelöst. Zu dieser Lösung werden 1.77 g (7.65 mmol) (-)-Diaceton-2-keto-L-gulonsäure auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert. Die erzielten Ausbeuten und ee-Werte sind in Tabelle 1a angegeben.

### Tabelle 1a

| Lösungsmittel | EtOH | Toluol | MTBE | EtOAc | CH$_3$CN | MeOH |
|---|---|---|---|---|---|---|
| Menge (ml) | 60 | 75 | 90 | 40 | 75 | 45 |
| ee (S) (%) | 69,5 | 35,2 | 17,6 | 38,6 | 42,8 | 57,3 |
| Ausbeute (%) | 33,4 | 40,0 | 56,0 | 37,0 | 37,0 | 31,0 |
| EtOH = Ethanol, MTBE = Methyl-tert butylether, EtOAc = Essigsäureethylester, MeOH= Methanol | | | | | | |

## Vergleichsbeispiel 1b

Durch Umsetzung des racemischen 3-Aminopentannitrils mit (-)-Diaceton-2-keto-L-gulonsäure in Analogie zu Beispiel 1 in Ethanol als Lösungsmittel wird ein Niederschlag des diastereomeren Salzes erhalten. Dieser wird einer weiteren Umkristallisation unterzogen, wodurch die optische Reinheit und damit der ee-Wert des (S)-enantiomerenangereicherten 3-Aminopentannitrils weiter erhöht werden kann. Der ee(S)-Wert des zur Umkristallisation eingesetzten diastereomeren Salzes beträgt nach einer in Analogie zu Beispiel 1 durchgeführten Extraktion 59,6.

Zur Umkristallisation werden 1,5 g des diastereomeren Salzes in der jeweils in Tabelle 1b angegebenen Menge des jeweiligen Lösungsmittels bei Siedetemperatur gelöst. Die weitere Aufarbeitung des beim Erkalten ausfallenden Salzes erfolgt wie für Beispiel 1 beschrieben.

### Tabelle 1b

| Lösungsmittel | EtOH | CH$_3$CN | Toluol | MTBE | EtOAc | MeOH |
|---|---|---|---|---|---|---|
| Menge (ml) | 40 | 40 | 50 | 40 | 50 | 35 |
| ee (S) (%) | 67,8 | 65,6 | 61,5 | 61,3 | 64,2 | 82,4 |
| Ausbeute (%) | 93,3 | 94,7 | 98,0 | 98,7 | 94,4 | 68,0 |

## Vergleichsbeispiel 2:

### Racematspaltung unter Verwendung von (S)-Methoxyphenylessigsäure

1.50 g (15.3 mmol) 3-Aminopentannitril werden in der in Tabelle 2 angegebenen Menge des jeweiligen Lösungsmittels gelöst. Zu dieser Lösung werden 1.27 g (7.64 mmol) (S)-Methoxyphenylessigsäure auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Die erzielten Ausbeuten und ee-Werte sind in Tabelle 2 angegeben.

### Tabelle 2

| Lösungsmittel | EtOH | Toluol | MTBE | EtOAc | CH$_3$CN | MeOH |
|---|---|---|---|---|---|---|
| Menge (ml) | 60 | 40 | 45 | 30 | 15 | 15 |
| ee (S) (%) | 50,7 | 54,7 | 55,8 | 49,7 | 71,6 | 81,6 |
| Ausbeute (%) | 40,0 | 28,0 | 3,0 | 31,0 | 53,0 | 15,0 |

## Vergeichsbeispiel 3:

### Racematspaltung unter Verwendung von (S)-(+)-6-Methoxy-α-methyl- 2-naphthyl- essigsäure

1.50 g (15.3 mmol) 3-Aminopentannitril werden in 10 ml Ethanol gelöst. Zu dieser Lösung werden 1.76 g (7.64 mmol) (S)-(+)-6-Methoxy-α-methyl-2-naphthylessigsäure auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Es werden 0.60 g (40,0 %) (R)-3-Aminopentannitril als farblose Flüssigkeit erhalten. Der ee (R)-Wert beträgt 27,4 %.

### Vergleichsbeispiel 4:

### Racematspaltung unter Verwendung von (S)-(+)-2-(4-Isobutyl-phenyl)-propionsäure

1.50 g (15.3 mmol) 3-Aminopentannitril werden in 10 ml Ethanol gelöst. Zu dieser Lösung werden 1.58 g (7.64 mmol) (S)-(+)-2-(4-Isobutylphenyl)-propionsäure auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Es werden 0.78 g (52,0 %) (R)-3-Aminopentannitril als farblose Flüssigkeit erhalten. Der ee(R)-Wert beträgt 2,4 %.

### Beispiel 5:

### Racematspaltung unter Verwendung von (L)-N-Boc-Isoleucin

1.50 g (15.3 mmol) 3-Aminopentannitril werden in der in Tabelle 3a angegebenen Menge des verwendeten Lösungsmittels gelöst. Zu dieser Lösung werden 1.77 g (7.64 mmol) (L)-N-Boc-Isoleucin auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Die erzielten Ausbeuten und ee-Werte sind in Tabelle 3a angegeben.

### Tabelle 3a

| Lösungsmittel | EtOH | Toluol | MTBE | EtOAc | $CH_3CN$ |
|---|---|---|---|---|---|
| **Menge (ml)** | 10 | 15 | 15 | 15 | 15 |
| **ee (R) (%)** | 92,2 | 68,7 | 85,7 | 81,4 | 82,5 |
| **Ausbeute (%)** | 6,7 | 42,0 | 35,0 | 36,0 | 29,0 |

### Beispiel 5b:

Durch Umsetzung des racemischen 3-Aminopentannitrils mit (L)-N-Boc-Isoleucin in Analogie zu Beispiel 5 in Ethanol als Lösungsmittel wird ein Niederschlag des diastereomeren Salzes erhalten. Dieser wird einer weiteren Umkristallisation unterzogen, wodurch die optische Reinheit und damit der ee-Wert des (R)-enantiomerenangereicherten 3-Aminopentannitrils weiter erhöht werden kann. Der ee(R)-Wert des zur Umkristallisation eingesetzten diastereomeren Salzes beträgt nach einer in Analogie zu Beispiel 5 durchgeführten Extraktion 90,4.

Zur Umkristallisation werden 1,25 g des diastereomeren Salzes in der jeweils in Tabelle 3b angegebenen Menge des jeweiligen Lösungsmittels bei Siedetemperatur gelöst. Die weitere Aufarbeitung des beim Erkalten ausfallenden Salzes erfolgt wie für Beispiel 5 beschrieben.

### Tabelle 3b

| Lösungsmittel | EtOH | $CH_3CN$ | Toluol | MTBE | EtOAc | MeOH |
|---|---|---|---|---|---|---|
| **Menge (ml)** | 4 | 10 | 5 | 45 | 5 | 2 |
| **ee (R) (%)** | 98,5 | 98,3 | 95,3 | 98,7 | 96,8 | 98,0 |
| **Ausbeute (%)** | 34,9 | 32,2 | 80,6 | 69,8 | 56,4 | 29,5 |

**Beispiel 6:**

**Racematspaltung unter Verwendung von (L)-N-Boc-Isoleucin mit anschließender Fällung des enantiomerenangereicherten 3-Aminopentannitrilmethansulfonats**

33.05 g (336.7 mmol) 3-Aminopentannitril werden in 830 ml *tert*-Butylmethylether gelöst. Zu dieser Lösung werden 39.0 g (168.6 mmol) (L)-N-Boc-Isoleucin auf einmal zugegeben. Die Suspension wird 5 Minuten zum Sieden erhitzt. Nach dem Abkühlen auf 20°C wird das als Feststoff anfallende Produkt abfiltriert und zweimal mit je 50 ml *tert*-Butylmethylether gewaschen. Das Produkt wird einmal aus 1650 ml *tert*-Butylmethylether umkristallisiert. Nach dem Abkühlen auf 20°C wird der Feststoff abgesaugt und zweimal mit je 50 ml *tert*-Butylmethylether gewaschen.

Es werden 35.0 g eines farblosen Feststoffes erhalten.

Dieser wird in 700 ml *tert*-Butylmethylether suspendiert. Zu dieser Suspension werden anschließend 10.22 g (106.3 mmol) Methansulfonsäure tropfenweise zugegeben. Die Suspension wird 5 Minuten zum Sieden erhitzt. Nach dem Abkühlen auf 20°C wird der Feststoff abgesaugt, zweimal mit je 25 ml *tert*-Butylmethylether gewaschen und bei 50°C für 10 Stunden unter vermindertem Druck getrocknet.

Es werden 19.8 g (30,3 %) (R)-3-Aminopentannitrilmethansulfonat als farbloser Feststoff erhalten.

Zur Bestimmung der optischen Reinheit wird 1 g des erhaltenen Produkts in 20 ml 1N Natronlauge gelöst und die Lösung zweimal mit je 10 ml Dichlormethan extrahiert. Nach Entfernen des Lösungsmittels unter vermindertem Druck wird aus dem Rückstand der ee-Wert gaschromatographisch bestimmt. Der ee (R)-Wert beträgt 99,0 %.

**NMR (400 MHz, $D_2O$):** δ (ppm) = 1.00 (t, J = 7.5 Hz, 3H), 1.83 (dt, J = 7.3 / 7.3 Hz, 2H), 2.80 (s, 3H), 3.02 (d, J = 5.3 Hz, 2H), 3.63 (tt, J = 6.3 / 6.3 Hz, 1 H), 4.75 (s, 3H).

**Schmelzbereich:** 109-111 °C

| Elementaranalyse: | ber.: | C = 37.11 | gef.: | C = 37.3 |
|---|---|---|---|---|
| | ber.: | H = 7.27 | gef.: | H = 7.4 |
| | ber.: | N = 14.42 | gef.: | N = 14.1 |
| | ber.: | S = 16.48 | gef.: | S = 16.4 |

**Beispiel 7:**

**Racematspaltung unter Verwendung von (L)-N-Boc-Serin**

1.50 g (15.3 mmol) 3-Aminopentannitril werden in der in Tabelle 4 angegebenen Menge des verwendeten Lösungsmittels gelöst. Zu dieser Lösung werden 1.57 g (7.64 mmol) (L)-N-Boc-Serin auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Die erzielten Ausbeuten und ee-Werte sind in Tabelle 4 angegeben.

**Tabelle 4**

| Lösungsmittel | EtOH | Toluol | MTBE | EtOAc | $CH_3CN$ | MeOH |
|---|---|---|---|---|---|---|
| **Menge (ml)** | 10 | 60 | 30 | 60 | 60 | 8 |
| **ee (R) (%)** | 70,0 | 70,7 | 76,0 | 80,4 | 63,3 | 86,8 |
| **Ausbeute (%)** | 29,3 | 26,0 | 43,0 | 30,0 | 32,0 | 55,0 |

**Vergleichsbeispiel 8:**

**Racematspaltung unter Verwendung von (S)-(-)-Phenylcarbamoylmilchsäure**

1.50 g (15.3 mmol) 3-Aminopentannitril werden in 10 ml Ethanol gelöst. Zu dieser Lösung werden 1.60 g (7.64 mmol) (S)-(-)-Phenylcarbamoylmilchsäure auf einmal zugegeben. Die Suspension wird 5 Minuten am Rückfluss erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Es werden 0.16 g (10,6%) (R)-3-Aminopentannitril als farblose Flüssigkeit erhalten. Der ee (R)-Wert beträgt 23,1%.

**Vergleichsbeispiel 9:**

**Racematspaltung unter Verwendung von (-)-O,O'-Dibenzoyl-(L)-weinsäure**

1.50 g (15.3 mmol) 3-Aminopentannitril werden in 20 ml Ethanol gelöst. Zu dieser Lösung werden 2.74 g (7.64 mmol) (-)-O,O'-Dibenzoyl-(L)-weinsäure auf einmal zugegeben. Die Suspension wird 5 Minuten am Rückfluss erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Es werden 0.84 g (56,0 %) (R)-3-Aminopentannitril als farblose Flüssigkeit erhalten. Der ee (R)-Wert beträgt 4,4 %.

**Vergleichsbeispiel 10:**

**Racematspaltung unter Verwendung von (-)-Di-O-p-toluyl-(L)-weinsäure**

1.50 g (15.3 mmol) 3-Aminopentannitril werden in 15 ml Ethanol gelöst. Zu dieser Lösung werden 2.95 g (7.64 mmol) (-)-Di-O-p-toluyl-(L)-weinsäure auf einmal zugegeben. Die Suspension wird 5 Minuten zu Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Es werden 1.04 g (69,3 %) (R)-3-Aminopentannitril als farblose Flüssigkeit erhalten. Der ee (R)-Wert beträgt 17,4 %.

**Vergleichsbeispiel 11:**

**Racematspaltung unter Verwendung von (-)-Menthoxyessigsäure**

1.50 g (15.3 mmol) 3-Aminopentannitril werden in 10 ml Ethanol gelöst. Zu dieser Lösung werden 1.64 g (7.64 mmol) (-)-Menthoxyessigsäure auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Es werden 0,30 g (20,0 %) (S)-3-Aminopentannitril als farblose Flüssigkeit erhalten. Der ee (S)-Wert beträgt 55,2 %.

**Vergleichsbeispiel 12:**

**Racematspaltung unter Verwendung von (S)-(-)-2-Pyrrolidinon-5-carbonsäure**

1.50 g (15.3 mmol) 3-Aminopentannitril werden in der in Tabelle 5 angegebenen Menge des verwendeten Lösungsmittels gelöst. Zu dieser Lösung werden 0,99 g (7.6 mmol) (S)-(-)-2-Pyrrolidinon-5-carbonsäure auf einmal zugegeben. Die Suspension wird 5 Minuten zum Sieden erhitzt. Nach dem Abkühlen auf 20°C wird das als Feststoff anfallende Produkte abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abdestilliert.

Die erzielten Ausbeuten und ee-Werte sind in Tabelle 5 angegeben.

**Tabelle 5**

| Lösungsmittel | EtOH | MTBE | EtOAc | CH$_3$CN |
|---|---|---|---|---|
| **Menge (ml)** | 15 | 15 | 15 | 15 |
| **ee (S) (%)** | 55,2 | 39,0 | 35,9 | 26,3 |
| **Ausbeute (%)** | 29,3 | 43,0 | 30,0 | 32,0 |

## Vergleichsbeispiel 13:

### Racematspaltung unter Verwendung von N-Acetyl-(L)-leucin

1.50 g (15.3 mmol) 3-Aminopentannitril werden in der in Tabelle 6a angegebenen Menge des verwendeten Lösungsmittels gelöst. Zu dieser Lösung werden 1.32 g (7.64 mmol) N-Acetyl-(L)-leucin auf einmal zugegeben. Die Suspension wird 5 Minuten zum Sieden erhitzt. Nach dem Abkühlen auf 20°C wird das als Feststoff anfallende Produkt abfiltriert und 10 Stunden unter vermindertem Druck bei 40°C getrocknet. Anschließend wird das Produkt in 20 ml 1N Natronlauge gelöst. Die wässrige Phase wird zweimal mit je 10 ml Dichlormethan extrahiert. Das Lösungsmittel der vereinigten organischen Phasen wird unter vermindertem Druck abfiltriert.

Die erzielten Ausbeuten und ee-Werte sind in Tabelle 6a angegeben.

**Tabelle 6a**

| Lösungsmittel | EtOH | Toluol | MTBE | EtOAc | CH$_3$CN | MeOH |
|---|---|---|---|---|---|---|
| **Menge (ml)** | 15 | 25 | 25 | 25 | 25 | 15 |
| **ee (R) (%)** | 66,4 | 46,7 | 50,7 | 42,4 | 41,2 | 71,1 |
| **Ausbeute (%)** | 46,0 | 47,0 | 50,0 | 48,0 | 45,0 | 25,0 |

## Vergleichsbeispiel 13b:

Durch Umsetzung des racemischen 3-Aminopentannitrils mit N-Acetyl-(L)-leucin in Analogie zu Beispiel 13 in Ethanol als Lösungsmittel wird ein Niederschlag des diastereomeren Salzes erhalten. Dieser wird einer weiteren Umkristallisation unterzogen, wodurch die optische Reinheit und damit der ee-Wert des (R)-enantiomerenangereicherten 3-Aminopentannitrils weiter erhöht werden kann. Der ee(R)-Wert des zur Umkristallisation eingesetzten diastereomeren Salzes beträgt nach einer in Analogie zu Beispiel 13 durchgeführten Extraktion 56,0.

Zur Umkristallisation werden 1,2 g diastereomeren Salzes in der jeweils in Tabelle 6b angegebenen Menge des jeweiligen Lösungsmittels bei Siedetemperatur gelöst. Die weitere Aufarbeitung des beim Erkalten ausfallenden Salzes erfolgt wie für Beispiel 13 beschrieben.

**Tabelle 6b**

| Lösungsmittel | EtOH | CH$_3$CN | MeOH |
|---|---|---|---|
| **Menge (ml)** | 20 | 60 | 8 |
| **ee (R) (%)** | 78,8 | 65,3 | 87,4 |
| **Ausbeute (%)** | 50,7 | 62,2 | 36,9 |

### Patentansprüche

**1.** Verfahren zur Gewinnung von enantiomerenangereichertem 3-Aminopentannitril oder dessen Salzen aus racemischem 3-Aminopentannitril durch

1) Umsetzung von racemischem 3-Aminopentannitril mit einer enantiomerenangereicherten organischen Säure unter Bildung zweier diastereomerer Salze aus dem 3-Aminopentannitril und der organischen Säure,
2) Abtrennung eines diastereomeren Salzes aus dem Reaktionsgemisch und
3) Überführung dieses diastereomeren Salzes in das enantiomerenangereicherte 3-Aminopentannitril oder dessen Salze

**dadurch gekennzeichnet, dass** (L)-N-Boc-alanin, (L)-N-Boc-asparaginsäure, (L)-N-Boc-Histidin, (L)-N-Boc-Isoleucin, (L)-N-Boc-Leucin, (L)-N-Boc-Methionin, (L)-N-Boc-phenylalanin, (L)-N-Boc-Prolin, (L)-N-Boc-Serin, (L)-N-Boc-Threonin, (L)-N-Boc-Tyrosin, (L)-N-Boc-Valin, oder ihre jeweiligen Enantiomere als enantiomeren-angereicherte organische Säuren eingesetzt werden.

2.  Verfahren nach Anspruch 1 zur Gewinnung von (R)-enantiomerenangereichertem 3-Aminopentannitril oder dessen Salzen, **dadurch gekennzeichnet, dass** als enantiomerenangereicherte organische Säure, (L)-N-Boc-Isoleucin oder (L)-N-Boc-Serin eingesetzt werden.

3.  Verfahren nach einem der Ansprüche 1 bis 2, **dadurch gekennzeichnet, dass** die Umsetzung in Schritt 1) bei einer Temperatur im Bereich von 0°C bis zur Zersetzungstemperatur der Reaktionsteilnehmer durchgeführt wird.

4.  Verfahren nach einem oder mehreren der Ansprüche 1 bis 3, **dadurch gekennzeichnet, dass** die Umsetzung in Schritt 1) in Gegenwart eines polaren oder unpolaren organischen Lösungsmitteln aus der Gruppe Diethylether, *tert*-Butylmethylether, Tetrahydrofuran, Methanol, Ethanol, n-Propanol, iso-Propanol, Toluol, Acetonitril und Essigsäureethylester durchgeführt wird.

5.  Verfahren nach einem oder mehreren der Ansprüche 1 bis 4, **dadurch gekennzeichnet, dass** 0.1-1 1 Äquivalente der enantiomerenangereicherten organischen Säure eingesetzt werden.

6.  Verfahren nach einem oder mehreren der Ansprüche 1 bis 5, **dadurch gekennzeichnet, dass** die Überführung des diastereomeren Salzes in das enantiomerenangereicherte 3-Aminopentannitril in Schritt 3) durch Umsetzung des diastereomeren Salzes mit Alkali- oder Erdalkalihydroxiden, Alkali- oder Erdalkalicarbonaten erfolgt.

7.  Verfahren nach einem oder mehreren der Ansprüche 1 bis 6 zur Gewinnung eines Salzes des enantiomerenangereicherten 3-Aminopentannitrils, **dadurch gekennzeichnet, dass** die Gewinnung des Salzes des enantiomerenangereicherten 3-Aminopentannitrils in Schritt 3) durch Umsetzung des diastereomeren Salzes mit einer Säure aus der Gruppe Salzsäure, Jodwasserstoffsäure, Bromwasserstoffsäure, Flusssäure, Schwefelsäure, Salpetersäure, Perchlorsäure, Phosphorsäure oder Methansulfonsäure unter Bildung des enantiomerenangereicherten 3-Aminopentannitril-Salzes dieser Säure erfolgt.

8.  Verfahren nach einem oder mehreren der Ansprüche 1 bis 7, **dadurch gekennzeichnet, dass** die Abtrennung des diastereomeren Salzes aus dem Reaktionsgemisch in Schritt 2) durch Abfiltrieren erfolgt.

9.  Verfahren nach Anspruch 8, **dadurch gekennzeichnet, dass** das nach dem Abfiltrieren des diastereomeren Salzes in Schritt 2) verbleibende Reaktionsgemisch zwecks Gewinnung des zweiten diastereomeren Salzes aufgearbeitet wird.

10. Verfahren nach einem oder mehreren der Ansprüche 1 bis 8, **dadurch gekennzeichnet, dass** das in Schritt 2) aus dem Reaktionsgemisch abgetrennte diastereomere Salz vor Durchführung von Schritt 3) zusätzlich einer oder mehrerer Umkristallisationen unterzogen wird.

**Claims**

1.  Process for the obtainment of enantiomerically enriched 3-aminopentanenitrile or its salts from racemic 3-aminopentanenitrile by

    1) reaction of racemic 3-aminopentanenitrile with an enantiomerically enriched organic acid with the formation of two diastereomeric salts of 3-aminopentanenitrile and the organic acid,
    2) separation of one diastereomeric salt from the reaction mixture and
    3) conversion of this diastereomeric salt into the enantiomerically enriched 3-aminopentanenitrile or its salts, **characterized in that** the enantiomerically enriched organic acids employed are (L)-N-Boc-alanine, (L)-N-Boc-aspartic acid, (L)-N-Boc-histidine, (L)-N-Boc-isoleucine, (L)-N-Boc-leucine, (L)-N-Boc-methionine, (L)-N-Boc-phenylalanine, (L)-N-Boc-proline, (L)-N-Boc-serine, (L)-N-Boc-threonine, (L)-N-Boc-tyrosine, (L)-N-Boc-valine, or their respective enantiomers.

**2.** Process according to Claim 1 for the obtainment of (R)-enantiomerically enriched 3-aminopentanenitrile or its salts, **characterized in that** the enantiomerically enriched organic acid employed is (L)-N-Boc-isoleucine or (L)-N-Boc-serine.

**3.** Process according to either of Claims 1 or 2, **characterized in that** the reaction in step 1) is carried out at a temperature in the range from 0°C up to the decomposition temperature of the reactants.

**4.** Process according to one or more of Claims 1 to 3, **characterized in that** the reaction in step 1) is carried out in the presence of polar or non-polar organic solvents from the group of diethyl ether, *tert*-butyl methyl ether, tetrahydrofuran, methanol, ethanol, n-propanol, iso-propanol, toluene, acetonitrile and ethyl acetate.

**5.** Process according to one or more of Claims 1 to 4, **characterized in that** 0.1 - 1 equivalent of the enantiomerically enriched organic acid is employed.

**6.** Process according to one or more of Claims 1 to 5, **characterized in that** the conversion of the diastereomeric salt into the enantiomerically enriched 3-aminopentanenitrile in step 3) is carried out by reaction of the diastereomeric salt with alkali metal or alkaline earth metal hydroxides, alkali metal or alkaline earth metal carbonates.

**7.** Process according to one or more of Claims 1 to 6 for the obtainment of a salt of the enantiomerically enriched 3-aminopentanenitrile, **characterized in that** the obtainment of the salt of the enantiomerically enriched 3-aminopentanenitrile in step 3) is carried out by reaction of the diastereomeric salt with an acid from the group of hydrochloric acid, hydriodic acid, hydrobromic acid, hydrofluoric acid, sulphuric acid, nitric acid, perchloric acid, phosphoric acid or methanesulphonic acid, with formation of the enantiomerically enriched 3-aminopentanenitrile salt of this acid.

**8.** Process according to one or more of Claims 1 to 7, **characterized in that** the separation of the diastereomeric salt from the reaction mixture in step 2) is carried out by filtering off.

**9.** Process according to Claim 8, **characterized in that** the the reaction mixture which remains after filtering the diastereomeric salt in step 2) is worked up for the purpose of obtainment of the second diastereomeric salt.

**10.** Process according to one or more of Claims 1 to 8, **characterized in that** the diastereomeric salt separated from the reaction mixture in step 2) is additionally subjected to one or more recrystallizations before carrying out step 3).

**Revendications**

**1.** Procédé pour l'obtention de 3-aminopentane-nitrile enrichi en énantiomère ou de ses sels à partir de 3-aminopentane-nitrile racémique, par

1) mise en réaction de 3-aminopentane-nitrile racémique avec un acide organique enrichi en énantiomère, avec formation de deux sels diastéréoisomères à partir du 3-aminopentanenitrile et de l'acide organique,
2) séparation d'un sel diastéréoisomère à partir du mélange réactionnel et
3) conversion de ce sel diastéréoisomère en le 3-aminopentane-nitrile enrichi en énantiomère ou ses sels,

**caractérisé en ce qu'**on utilise comme acides organiques enrichis en énantiomère la (L)-N-Boc-alanine, l'acide (L)-N-Boc-aspartique, la (L)-N-Boc-histidine, la (L)-N-Boc-isoleucine, la (L)-N-Boc-leucine, la (L)-N-Boc-méthionine, la (L)-N-Boc-phénylalanine, la (L)-N-Boc-proline, la (L)-N-Boc-sérine, la (L)-N-Boc-thréonine, la (L)-N-Boc-tyrosine, la (L)-N-Boc-valine, ou leurs énantiomères respectifs.

**2.** Procédé selon la revendication 1, pour l'obtention de 3-aminopentane-nitrile riche en (R)-énantiomère ou de ses sels, **caractérisé en ce qu'**on utilise comme acide organique enrichi en énantiomère la (L)-N-Boc-isoleucine ou la (L)-N-Boc-sérine.

**3.** Procédé selon la revendication 1 ou 2, **caractérisé en ce qu'**on effectue la réaction dans l'étape 1) à une température dans la plage de 0 °C à la température de décomposition des partenaires réactionnels.

**4.** Procédé selon une ou plusieurs des revendications 1 à 3, **caractérisé en ce qu'**on effectue la réaction dans l'étape 1) en présence d'un solvant organique polaire ou non polaire, choisi dans le groupe constitué par l'oxyde d'éthyle, l'oxyde de méthyle et de tert-butyle, le tétrahydrofuranne, le méthanol, l'éthanol, le n-propanol, l'isopropanol, le toluène, l'acétonitrile et l'acétate d'éthyle.

5. Procédé selon une ou plusieurs des revendications 1 à 4, **caractérisé en ce qu'**on utilise 0,1-1 équivalent de l'acide organique enrichi en énantiomère.

6. Procédé selon une ou plusieurs des revendications 1 à 5, **caractérisé en ce que** la conversion du sel diastéréoisomère en le 3-aminopentane-nitrile enrichi en énantiomère dans l'étape 3) s'effectue par réaction du sel diastéréoisomère avec des hydroxydes de métaux alcalins ou alcalino-terreux, des carbonates de métaux alcalins ou alcalino-terreux.

7. Procédé selon une ou plusieurs des revendications 1 à 6, pour l'obtention d'un sel du 3-aminopentane-nitrile enrichi en énantiomère, **caractérisé en ce que** l'obtention du sel du 3-aminopentane-nitrile enrichi en énantiomère dans l'étape 3) s'effectue par réaction du sel diastéréoisomère avec un acide choisi dans le groupe constitué par les acides chlorhydrique, iodhydrique, bromhydrique, fluorhydrique, sulfurique, nitrique, perchlorique, phosphorique et méthanesulfonique, avec formation du sel de 3-aminopentane-nitrile de cet acide, enrichi en énantiomère.

8. Procédé selon une ou plusieurs des revendications 1 à 7, **caractérisé en ce que** la séparation du sel diastéréoisomère à partir du mélange réactionnel dans l'étape 2) s'effectue par filtration.

9. Procédé selon la revendication 8, **caractérisé en ce que** le mélange réactionnel restant après la séparation du sel diastéréoisomère par filtration dans l'étape 2) est soumis à un traitement final pour la récupération du deuxième sel diastéréoisomère.

10. Procédé selon une ou plusieurs des revendications 1 à 8, **caractérisé en ce que** le sel diastéréoisomère séparé du mélange réactionnel dans l'étape 2) est en outre soumis à une ou plusieurs recristallisations avant l'exécution de l'étape 3).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4260556 A
- US 4496474 A
- EP 0449297 A
- US 5902883 A
- US 4072698 A
- WO 2004002924 A

**In der Beschreibung aufgeführte Nicht-Patentliteratur**

- **Jacques, Jean et al.** Enantiomers, Racemates, and Resolutions. Wiley-Interscience Publication, 1981, 252-399
- *J. Nat. Prod.,* 2002, vol. 65, 29-31